(19) Europäisches Patentamt
European Patent Office
Office européen des brevets

(11) Veröffentlichungsnummer: **0 248 365**
**A1**

# (12) EUROPÄISCHE PATENTANMELDUNG

(21) Anmeldenummer: **87107816.8**

(22) Anmeldetag: **29.05.87**

(51) Int. Cl.[4]: **C07D 233/14** , C07D 233/18 , C07D 233/22 , D06M 13/46

(30) Priorität: **05.06.86 DE 3618944**

(43) Veröffentlichungstag der Anmeldung: **09.12.87 Patentblatt 87/50**

(84) Benannte Vertragsstaaten: **AT BE CH DE ES FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Ploog, Uwe, Dr.**
**Haydnweg 6**
**D-5657 Haan(DE)**
Erfinder: **Uphues, Günter**
**Robert Koch Strasse 45**
**D-4019 Monheim(DE)**

(54) **Quartäre 2-Alkylimidazoliniumsalze, Verfahren zu deren Herstellung und deren Verwendung.**

(57) Durch Umsetzung von entsprechenden 2-Alkylimidazolinen mit Ethylenoxid oder Propylenoxid werden quartäre 2-Alkyl-2-imidazoliniumsalze der allgemeinen Formel (I)

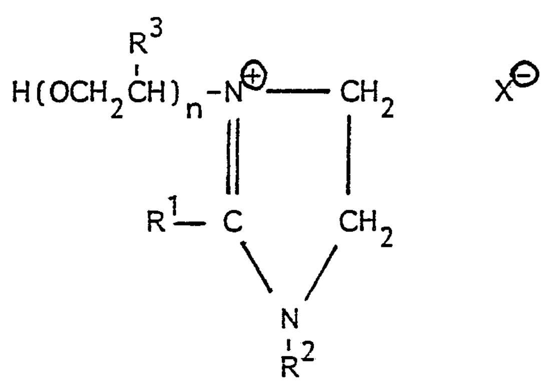

(I)

in der
$R^1$ für einen geradkettigen oder verweigten, substituierten oder unsubstituierten Alkyl-oder Alkenylrest mit 7 bis 21 C-Atomen,
$R^2$ für einen Rest der Strukturen

EP 0 248 365 A1

$$-(CH_2-\overset{R^4}{\overset{|}{C}}H-O)_m H \quad \text{oder}$$

$$-CH_2-CH_2-NH-\overset{O}{\overset{\|}{C}}-R^1 ,$$

$R^3$ für Wasserstoff oder Methyl,
$R^4$ für Wasserstoff oder Methyl,
$X^{\ominus}$ für das Anion einer nicht oxidierend und nicht korrosiv wirkenden anorganischen Säure oder einer organischen Mono-oder Polycarbonsäure und
n statistisch für ganze oder gebrochene Zahlen im Bereich von 1 bis 20 und
m für ganze Zahlen im Bereich von 1 bis 3
stehen,
erhalten, die für die Avivage von Textilien eingesetzt werden können.

# Quartäre 2-Alkylimidazoliniumsalze, Verfahren zu deren Herstellung und deren Verwendung

Die Erfindung betrifft quartäre 2-Alkylimidazoliniumsalze, ein Verfahren zu deren Herstellung und deren Verwendung.

Es ist seit langem bekannt, daß quartäre Tetraalkylammoniumverbindungen mit 2 langkettigen und 2 kurzkettigen Alkylresten, beispielsweise 2 $C_{16}$-$C_{18}$-Alkylresten und 2 Methylresten, gewaschenen Textilien einen weichen Griff verleihen, wenn sie beim Wäschewaschen dem letzten Spülbad zugesetzt werden. Diese Textilweichmacher werden in großem Umfang sowohl von gewerblichen Wäschereien als auch bei der Wäsche im Haushalt eingesetzt. Es wird angenommen, daß diese kationischen quartären Ammoniumverbindungen wegen ihrer positiven Ladung leicht auf das Textilsubstrat aufziehen.

Der am häufigsten verwendete Textilweichmacher dieser Verbindungsklasse ist Distearyldimethylammoniumchlorid (siehe S. Billenstein et al. J. Am. Oil Chem. Soc. 61 (1984) S. 353 bis 357). Textilweichmacher dieses Typs besitzen zwar ausgezeichnete weichmachende Eigenschaften, weisen aber auch einige Nachteile auf. So besitzen die damit behandelten Gewebe eine gegenüber den unbehandelten Geweben verminderte Saugfähigkeit, was vom Verbraucher zum Beispiel bei Kleidungsstücken, die im Kontakt mit der Haut getragen werden, und bei Frottierhandtüchern als unangenehm empfunden werden kann. Derartige Textilweichmacher lassen sich bei der Wäsche häufig nicht restlos vom Gewebe wieder entfernen, so daß es - auch bei richtiger Dosierung - mitunter zu einer Akkumulierung der weichmachenden Wirkstoffe kommt, womit ebenfalls eine verminderte Saugwirkung der Textilien verbunden ist. Ein weiterer gravierender Nachteil dieser Textilweichmacher ist die durch das Chloridanion verursachte korrosive Wirkung gegenüber Metalloberflächen.

Es hat nicht an Versuchen gefehlt, die Eigenschaften von Textilweichmachern auf Basis von quartären Ammoniumverbindungen zu verbessern. So ist es aus der US-PS 3,636,114 bekannt, daß durch die Verwendung von quartären Ammoniumverbindungen mit 2 langkettigen 2-Hydroxyalkylresten die Saugfähigkeit der behandelten Textilien verbessert werden kann. Aus der DE-OS 22 56 234 sind quartäre Ammoniumverbindungen mit 2 langkettigen 2-Hydroxy-3-alkoxypropyl-Gruppen bekannt. Aus der DE-AS 16 19 043 sind Weichmachungsmittel bekannt, die quartäre Ammoniumverbindungen mit nur einem langen Alkylrest und 3 kurzen Alkylresten enthalten, wobei die drei kurzen Alkylreste Hydroxyl-und Ethergruppen enthalten können. In der niederländischen Patentanmeldung 86/08958 und der US-PS 3,591,405 sind quartäre Ammoniumverbindungen beschrieben, deren hydrophobe Reste Hydroxyalkylgruppen oder über Ethoxygruppen mit dem Stickstoff verknüpfte Alkylgruppen darstellen. Diese Lösungsversuche konnten aber nicht überzeugen, weil entweder die modifizierten quartären Ammoniumverbindungen präparativ schlecht zugänglich sind und daher für eine praktische Anwendung nicht in Frage kommen, oder weil die weichmachende Wirkung zu gering ist.

Der vorliegenden Erfindung lag daher die Aufgabe zugrunde, neue quartäre Ammoniumverbindungen bereitzustellen, die in einfacher Weise aus leicht zugänglichen Ausgangsmaterialien erhalten werden können. Die neuen Verbindungen sollten den damit behandelten Textilien bei guter weichmachender Wirkung die Saugfähigkeit erhalten und sich rückstandslos bei der Wäsche der Textilien entfernen lassen. Weiterhin sollten sie keine korrodierende Wirkung gegenüber Metalloberflächen aufweisen.

Die Erfindung betrifft daher quartäre 2-Alkylimidazoliniumsalze der allgemeinen Formel (I)

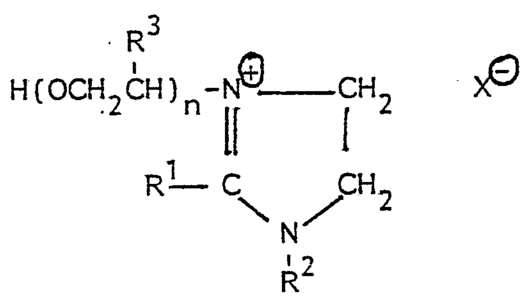

(I)

in der

$R^1$ für einen geradkettigen oder verzweigten, substituierten oder unsubstituierten Alkyl-oder Alkenylrest mit 7 bis 21 C-Atomen,

$R^2$ für einen Rest der Strukturen

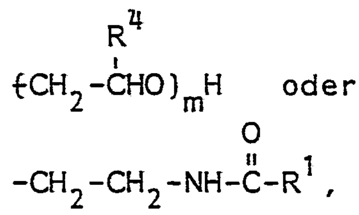

oder

$R^3$ für Wasserstoff oder Methyl,
$R^4$ für Wasserstoff oder Methyl,
X⊖ für das Anion einer nicht oxidierend und nicht korrosiv wirkenden anorganischen Säure oder einer organischen Mono-oder Polycarbonsäure und
n statistisch für ganze oder gebrochene Zahlen im Bereich von 1 bis 20 und
m für ganze Zahlen im Bereich von 1 bis 3
stehen.

Die Erfindung betrifft außerdem ein Verfahren zur Herstellung von quartären 2-Alkyl-2-imidazoliniumsalzen der allgemeinen Formel (I)

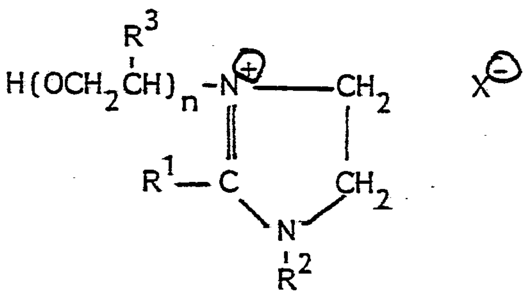

(I)

das dadurch gekennzeichnet ist, daß man Imidazoline der allgemeinen Formel (II)

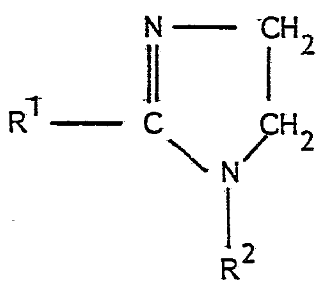

(II)

in der $R^1$ und $R^2$ die für die Formel (I) angegebene Bedeutung haben, in Gegenwart von 1 bis 1,1 Äquivalenten einer Säure der Formel HX (III) in der X die für die Formel (I) angegebene Bedeutung hat, und gegebenenfalls in Gegenwart von Wasser oder eines Gemisches aus Wasser und einem polaren organischen Lösungsmittel in an sich bekannter Weise mit Ethylenoxid oder Propylenoxid umsetzt und die entstandenen Produkte gegebenenfalls auf an sich bekannten Wegen isoliert und/oder reinigt.

Die Erfindung betrifft außerdem die Verwendung quartärer 2-Alkyl-2-imidazoliniumsalze der allgemeinen Formel (I)

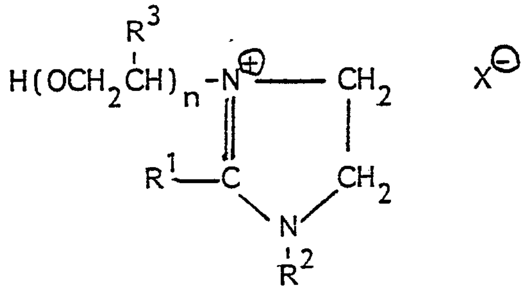

(I)

zur Avivage von Textilien.

In der oben angegebenen allgemeinen Formel (I) kann $R^1$ für einen geradkettigen oder verzweigten, substituierten oder unsubstituierten Alkyl-oder Alkenylrest mit 7 bis 21 C-Atomen stehen. Als geeignete Alkylreste sind also beispielsweise die n-Alkylreste aus der Gruppe Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl oder Uneicosyl oder ihre einfach oder mehrfach verzweigten Homologen anzusehen. Dabei werden in den erfindungsgemäßen Imidazoliniumsalzen der allgemeinen Formel (I) bevorzugt solche Substituenten $R^1$ angetroffen, die aus Fettsäuren natürlicher Herkunft stammen. Als solche sind insbesondere geradkettige oder verzweigte unsubstituierte Alkyl-oder Alkenylreste mit 7 bis 17 C-Atomen aus der oben genannten Gruppe anzusehen, wobei die geradkettigen Alkylreste mit 7 bis 17 C-Atomen besonders hervorzuheben sind. Als besonders vorteilhaft sind dabei Verbindungen der allgemeinen Formel (I) anzusehen, in der $R^1$ für einen Alkylrest aus der Gruppe n-Heptyl, n-Nonyl, n-Undecyl, n-Pentadecyl, n-Heptadecyl, n-Heptadec-8-enyl, n-Heptadec-8,11-dienyl oder deren Mischungen steht.

Als ebenfalls unter die erfindungsgemäßen quartären 2-Alkyl-2-imidazoliniumsalze der allgemeinen Formel (I) fallend sind auch Verbindungen anzusehen, in denen $R^1$ für einen geradkettigen oder verzweigten Alkylrest mit 7 bis 17 C-Atomen steht, der mit einer oder mehreren Hydroxylgruppen substituiert ist. Als solche Reste sind Alkylreste $R^1$ anzusehen, die sich von Basisfettsäuren mit einem OH-Substituenten ableiten. Dadurch wird eine gegenüber den schon ohnehin gut löslichen Verbindungen der allgemeinen Formel (I) mit $R^1$ = Alkyl deutlich verbesserte Wasserlöslichkeit der Verbindungen erreicht, deren gute Wasserlöslichkeit Voraussetzung für ihre optimale Verwendung ist.

In der oben angegebenen allgemeinen Formel (I) kann der Substituent $R^2$ am Stickstoffatom in der 1-Position des fünfgliedrigen heterocyclischen Ringes für einen der beiden Reste der nachfolgenden Strukturen stehen:

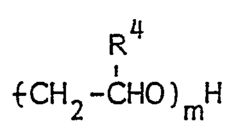

oder

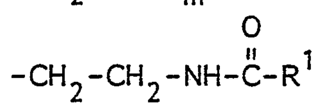

In diesen Strukturen steht $R^4$ für Wasserstoff oder Methyl und m für ganze Zahlen im Bereich von 1 bis 3, so daß geeignete Reste, in diesem Fall der Hydroxyethylrest, der 2-Hydroxypropylrest, der Ethoxyhydroxyethylrest sowie der bis-(Ethoxy-)hydroxyethylrest sind, von welchen der Hydroxyethylrest besonders bevorzugt ist.

In der allgemeinen Formel (I) für die erfindungsgemäßen quartären 2-Alkyl-2-imidazoliniumsalze steht X für das Anion einer nicht oxidierend und nicht korrosiv wirkenden anorganischen Säure oder einer organischen Mono-oder Polycarbonsäure. Als anorganische, nicht oxidierend und nicht korrosiv wirkende Säuren, deren Anion $X^\ominus$ repräsentiert, sind dabei phosphorige Säure, ortho-Phosphorsäure, Schwefelsäure und deren Partialester, beispielsweise Schwefelsäuremonomethylester, zu nennen. Bevorzugt steht jedoch $X^\ominus$ für das Anion einer Alkancarbonsäure mit 0 bis 17 C-Atomen im Alkylrest, das Anion einer Monohydroxyalkancarbonsäure mit 1 bis 17 C-Atomen im Alkylrest oder das Anion einer $\alpha,\omega$-Dicarbonsäure mit 0 bis 8 Methylengruppen, wobei die Methylengruppen einen Hydroxysubstituenten tragen können, oder das Anion der Fumar-, Malein-oder Citronensäure. Außer von den bereits explizit genannten Säuren kann sich das Anion $X^\ominus$ beispielsweise von Alkancarbonsäure aus der Gruppe Ameisensäure, Essigsäure, Propionsäure,

Buttersäure, Valeriansäure, Capronsäure, Oenanthsäure, Caprylsäure, Pelargonsäure, Caprinsäure, Undecansäure, Laurinsäure, Myristinsäure, Palmitinsäure und Stearinsäure sowie den Isomeren dieser Carbonsäuren von Monohydroxyalkancarbonsäuren wie Glykolsäure, Milchsäure, $\beta$-Hydroxypropionsäure oder 12-Hydroxystearinsäure ableiten.

$X^{\ominus}$ kann jedoch auch für das Anion einer $\alpha,\omega$-Dicarbonsäure aus der Gruppe Oxalsäure, Malonsäure, Bernsteinsäure, Glutarsäure, Adipinsäure, Pimelinsäure, Suberinsäure, Azelainsäure, Sebacinsäure, Nonandicarbonsäure oder Decandicarbonsäure stehen. Weitere geeignete Anionen $X^{\ominus}$ stammen von $\alpha,\omega$-Dicarbonsäure der oben genannten Gruppe ab, in denen die Methylengruppen einen Hydroxysubstituenten tragen. Als Anionen aus dieser Gruppe sind beispielsweise die Anionen der Äpfelsäure und der Weinsäure zu nennen.

Aus der großen Gruppe der oben genannten Anionen $X^{\ominus}$ sind in den Verbindungen der allgemeinen Formel (I) bevorzugt die Anionen $X^{\ominus}$ der Milchsäure oder der Essigsäure.

In der oben genannten allgemeinen Formel (I) für die erfindungsgemäßen quartären 2-Alkyl-2-imidazoliniumsalze trägt das Ammonium-Stickstoffatom in der 3-Position des heterocyclischen, fünfgliedrigen Ringes einen Substituenten der Formel $-(CHR^3-CH_2-O)_nH$ in dem n für einen statistischen Wert für die Zahl der Ethoxygruppen steht, der kontinuierlich im gesamten Bereich aller ganzen oder gebrochenen Zahlen von 1 bis 20 liegen kann. Die Zahl der Ethoxy-oder Propoxygruppen richtet sich dabei nach der Einsatzmenge des für die Quaternierung gemäß dem unten näher beschriebenen erfindungsgemäßen Verfahren verwendeten Olefinepoxids. Im Rahmen dieses Verfahrens wird - wie unten näher beschrieben - in den seltensten Fällen ein einheitliches Produkt mit einer bestimmten, ganzen Zahl n an Alkoxygruppen, sondern mehrheitlich eine ganze Palette unterschiedlich oxalkylierter Verbindungen nebeneinander gebildet.

In der obigen allgemeinen Formel (I) kann demzufolge $R^3$ für Wasserstoff oder einen Methylrest stehen, je nachdem, ob zur Quaternierung Ethylenoxid oder Propylenoxid verwendet wurde.

Bevorzugt steht $R^3$ in der obigen allgemeinen Formel (I) für Wasserstoff.

Die für die Herstellung der erfindungsgemäßen quartären 2-Alkylimidazoliniumsalze der allgemeinen Formel (I) eingesetzten 2-Alkylimidazoline der allgemeinen Formel (II) stellen bekannte Substanzen dar, die über bekannte Verfahren der organischen Synthese erhalten werden können (siehe beispielsweise US-PS 4 058 488 und US-PS 4 212 983).

Zur Herstellung der Imidazoline der allgemeinen Formel (II) kondensiert man zweckmäßigerweise Fettsäuren der allgemeinen Formel (III)

$R^1$-COOH (III)

in der $R^1$ für einen geradkettigen oder verzweigten, substituierten oder unsubstituierten Alkyl-oder Alkenylrest mit 7 bis 21 C-Atomen steht, mit einem Aminoalkylaminderivat der allgemeinen Formel (IV)

$H_2N-CH_2-CH_2-NH-R^{2'}$ (IV)

in der $R^{2'}$ für einen Rest der Strukturen

$$CH_2-\overset{R^4}{\overset{|}{C}}H-OH \quad \text{oder}$$

$$CH_2-CH_2-NH_2$$

steht, wobei $R^4$ Wasserstoff oder Methyl bedeutet.

Als Fettsäuren (III) kommen dabei synthetische oder aus nativen Quellen zugängliche Fettsäuren mit 8 bis 22 C-Atomen in Betracht. Bevorzugt werden für diese Umsetzung Fettsäuren der allgemeinen Formel (III) verwendet, in denen $R^1$ für einen geradkettigen oder verzweigten, unsubstituierten Alkyl-oder Alkenylrest mit 7 bis 17 C-Atomen steht. Andererseits sind auch solche Fettsäuren der allgemeinen Formel (III) verwendbar, in denen $R^1$ für einen geradkettigen oder verzweigten, mit einer oder mehreren Hydroxylgruppen substituierten Alkylrest mit 7 bis 17 C-Atomen steht. Vorzugsweise werden geradkettige Fett-und Hydroxyfettsäuren eingesetzt.

Beispiele für Fettsäuren, die mit Vorteil für die Herstellung der Imidazoline der allgemeinen Formel (II) eingesetzt werden können, sind Caprylsäure, Caprinsäure, Laurinsäure, Myristinsäure, Palmitinsäure, Stearinsäure, Ölsäure, Linolensäure, Ricinolsäure, 12-Hydroxystearinsäure, und 9,10-Dihydroxystearinsäure. Zur Herstellung der Imidazoline (II) können die genannten Fettsäuren einzeln oder im Gemisch verwendet werden. In der Regel werden für die Herstellung der Imidazoline (II) Fettsäuregemische eingesetzt wie sie bei der Spaltung von natürlich vorkommenden Fetten und Ölen anfallen. Auch einzelne Fraktionen solcher Fettsäuregemische können für die Synthese der Imidazoline (II) eingesetzt werden. Von besonderer Bedeutung sind hier die bei der Spaltung von Kokosöl anfallenden Gemische aus gesättigten und ungesättigten Fettsäuren, die häufig als "Kokosfettsäure" bezeichnet werden, und die daraus über die Hydrierung der vorhandenen Doppelbindungen erhaltenen Gemische aus gesättigten Fettsäuren, die auch als "hydrierte Kokosfettsäure" bezeichnet werden.

Als Reaktionspartner der oben genannten allgemeinen Formel (IV) wird in dem vorliegenden Verfahren Aminoethylethanolamin, Aminoethylpropanolamin oder Diethylentriamin verwendet. Das Molverhältnis der Verbindungen (III) : (IV), d.h. also von Fettsäure : Aminoalkylaminderivat kann dabei im Bereich von 1 : 1 bis 2 : 1 liegen. Dabei wird im Falle der Umsetzung einer Fettsäure mit Aminoethylethanolamin das Molverhältnis der Reaktionspartner im Bereich von 1 : 1 liegen, während bei der Umsetzung der Fettsäure mit Diethylentriamin das Molverhältnis 2 : 1 beträgt.

Die Kondensationsreaktion wird bei einer Reaktionstemperatur im Bereich von 150 bis 240°C, bevorzugt zwischen 150 und 200°C, durchgeführt. Anfallendes Reaktionswasser wird dabei laufend durch einfache oder azeotrope Destillation mit Hilfe eines geeigneten Lösungsmittels aus dem Reaktionsgemisch entfernt. Bei der Umsetzung von Fettsäure mit Aminoethylethanolamin wird in der Regel ein Lösungsmittel als Schleppmittel für das im Verlauf der Reaktion gebildete Wasser verwendet. Die Reaktion der Fettsäure mit Diethylentriamin kann gegebenenfalls auch in Abwesenheit eines Lösungsmittels durchgeführt werden. Die Lösungsmittelmenge ist dabei so zu bemessen, daß für das gesamte Reaktionsgemisch eine Endtemperatur von 150 bis 240°C, bevorzugt von 150 bis 200°C, erreicht werden kann. Als geeignete Lösungsmittel haben sich in diesem Zusammenhang insbesondere Toluol und Xylol bewährt. Gegen Ende der Reaktion wird der Druck im Reaktionsraum langsam vermindert, vorzugsweise bis zu einem Enddruck von 10 bis 20 mbar, um vorhandenes Lösungsmittel zu entfernen und noch abzuspaltendes Reaktionswasser auszutreiben.

In einem weiteren zweckmäßigen Verfahren zur Herstellung der Imidazoline der allgemeinen Formel (II) werden Ester der Fettsäuren der allgemeinen Formel (III) mit kurzkettigen Alkoholen, insbesondere Fettsäuremethylester oder -ethylester, als Ausgangsmaterial eingesetzt. Für die Fettsäurekomponente dieser Ester gelten die im Zusammenhang mit der vorstehend beschriebenen Imidazolinsynthese gemachten Angaben für die Fettsäuren sinngemäß. Auch in diesem Fall können einzelne Fettsäureester oder Gemische solcher Fettsäureester verwendet werden. Bevorzugt werden hier Estergemische eingesetzt, die einerseits aus der Umesterung von nativen Fetten und Ölen mit kurzkettigen Alkoholen oder andererseits aus der Veresterung von Spalt-Fettsäuregemischen mit kurzkettigen Alkoholen stammen können.

Der erste Reaktionsschritt besteht bei diesem Verfahren in einer Aminolyse der Fettsäureester, wobei der kurzkettige Alkohol des Esters freigesetzt und zur Verschiebung des Gleichgewichts aus dem Reaktionsgemisch abdestilliert wird. Die Aminolyse beginnt bereits bei Temperaturen ab 60 °C; für die nachfolgende Ringschlußreaktion unter Wasseraustritt sind wiederum Temperaturen im Bereich von 150 bis 240 °C erforderlich, so daß die Temperaturen der Gesamtreaktion im Bereich von 60 bis 240 °C liegen. Zur Beschleunigung der Aminolyse kann dem Gemisch der Ausgangsstoffe ein alkalischer Katalysator, beispielsweise Natriumhydroxyd, Kaliumhydroxid oder Natriummethylat zugesetzt werden. Die Reaktion kann in Abwesenheit von Lösungsmitteln durchgeführt werden. Es hat sich jedoch als zweckmäßig erwiesen, den zweiten Teil der Synthese so zu gestalten, daß das gebildete Wasser als Azeotrop mit einem geeigneten, nicht mit Wasser mischbaren organischen Lösungsmittel wie Toluol oder Xylol aus dem Reaktionsgemisch abdestilliert wird. In Analogie zur Synthese aus den freien Fettsäuren kann hier das Molverhältnis von Fettsäureester : Aminoalkylaminderivat im Bereich von 1 : 1 bis 2 : 1 liegen.

Die Herstellung der Imidazoline der allgemeinen Formel (II) kann schließlich auch direkt aus Fetten und Ölen natürlichen Ursprungs erfolgen, die bekanntlich Triglyceridgemische von Fettsäuren der allgemeinen Formel (III) darstellen. Geeignete natürlich vorkommende Triglyceridgemische sind beispielsweise Sojaöl und Talg. Von besonderer Bedeutung ist in diesem Zusammenhang Kokosöl. Die Rahmenbedingungen dieses Verfahrens sind praktisch dieselben wie die des unmittelbar vorstehend beschriebenen, mit der Ausnahme, daß freigesetztes Glycerin in der Regel nicht abgetrennt wird, sondern im Reaktionsgemisch verbleibt. Das Molverhältnis von Fettsäuretriglycerid : Aminoalkylaminderivat kann im Bereich von 1 : 3 bis 2 : 3 liegen.

Bei der Durchführung des erfindungsgemäßen Verfahrens zur Herstellung der quartären 2-Alkyl-2-imidazoliniumsalze der allgemeinen Formel (I) setzt man den Imidazolinen der allgemeinen Formel (II) pro Mol 1 bis 1,1 Äquivalente einer Säure HX zu, wobei für HX die im Zusammenhang mit den Verbindungen der Formel (I) gemachten Angaben gelten.

Die Säurezugabe kann dabei gegebenenfalls von einer Zugabe von Wasser begleitet sein. Wegen der Instabilität der Imidazoline im alkalischen Milieu wird das Wasser nach der Säurezugabe eingesetzt. Es hat sich als besonders vorteilhaft herausgestellt, anstelle von Wasser der Reaktionsmischung ein Gemisch von Wasser mit einem organischen polaren Lösungsmittel zuzusetzen. Als solche Lösungsmittel sind bevorzugt Alkohole mit 1 bis 6 C-Atomen verwendbar, beispielsweise Methanol, Ethanol, Propanol, n-Butanol, n-Hexanol oder deren verzweigtkettige Isomere. Besonders bevorzugt wird dabei ein Gemisch aus Wasser und i-Propanol im Volumenverhältnis 1 : 1.

In das Gemisch aus Imidazolin, Säure, Wasser und gegebenenfalls Lösungsmittel wird dann Ethylenoxid oder Propylenoxid eingeleitet. Das Molverhältnis Imidazolin : Alkylenoxid hat sich dabei im Bereich von 1 : 1 bis 20 : 1 als besonders vorteilhaft herausgestellt. Ein bevorzugtes Molverhältnis der Reaktionspartner liegt im Bereich von 1 : 3 bis 1 : 10. Als Alkylenoxid wird mit Vorteil Ethylenoxid verwendet.

Die Reaktion wird bei einer Temperatur von 80 bis 100°C über 2 bis 6 Stunden bei geringfügigem Überdruck durchgeführt. Der Druck liegt bevorzugt im Bereich von 1 bis 5 bar. Um den bei der Tensidherstellung üblichen Viskositätsanstieg aufzufangen ist es möglich, in dieser Reaktionsstufe gegebenenfalls noch Wasser zuzusetzen und damit die Viskosität so einzustellen, daß ein bei den Reaktionsbedingungen noch pumpbares Gemisch entsteht, das bei den Reaktionsbedingungen eine Viskosität von nicht mehr als 10 000 mPas aufweist.

Die in der Reaktionsmischung erhaltenen quartären 2-Alkyl-2-imidazoliniumsalze der allgemeinen Formel (I) können auf an sich bekannten Wegen isoliert und gereinigt werden. Dies geschieht beispielsweise durch Abdestillieren des Lösungsmittels und Isolieren bzw. Reinigen des erhaltenen Produktes über Kristallisation, Rekristallisation usw.

Die Verbindungen der allgemeinen Formel (I) stellen Kationtenside dar, die auf Baumwollgeweben eine überraschend gute Avivage-Wirkung zeigen. Gegenüber herkömmlichen Avivagemitteln besitzen die Verbindungen der allgemeinen Formel (I) den Vorteil, daß die im unbehandelten Gewebe vorhandene Saugfähigkeit durch die Avivagebehandlung mit den Verbindungen der allgemeinen Formel (I) überhaupt nicht beeinträchtigt wird. Die Verbindungen der allgemeinen Formel (I) werden erfindungsgemäß zur Avivage von Textilien, insbesondere von Baumwolltextilien, verwendet.

Die Verbindungen der allgemeinen Formel I lassen sich mit anderen Avivagemitteln, Antimikrobika, optischen Aufhellern, nichtionischen und anderen kationischen Tensiden und gegebenenfalls anderen üblichen Bestandteilen von Avivagemitteln problemlos zu Präparaten konfektionieren, die vorzugsweise flüssig sind. Die Einzelkomponenten solcher Präparate werden dabei mechanisch miteinander vermischt. Chemische Reaktionen treten bei der Herstellung der Präparate nicht ein. Die neuen Verbindungen können als Avivagemittel insbesondere in flüssigen Mitteln zu Wäschenachbehandlung im Spülbad des Waschprozesses bei der Hand-oder Maschinenwäsche und im Wäschetrockner eingesetzt werden. Dabei enthalten die Mittel 1 bis 40 Gewichtsprozent der Verbindungen der Formel I und wenigstens ein Verdünnungsmittel sowie gegebenenfalls sonstige übliche Zusatzstoffe für flüssige Wäschenachbehandlungsmittel.

Die Verwendung der erfindungsgemäßen Avivagemittel in Wäschereien und Haushaltungen zur Nachbehandlung von gewaschener Wäsche kann auf verschiedene Weise erfolgen, je nach der Wahl des Trocknungsverfahrens. Wird in einem Wäschetrockner getrocknet, so kann man die Wäsche direkt mit einer Wirkstoffdispersion besprühen oder den Wirkstoff über einen Träger mit dem Waschgut in Kontakt bringen, beispielsweise durch Besprühen der Innenwände des Trocknungsgerätes mit dem flüssigen Mittel vor dem Beladen des Geräts mit Wäsche, oder durch Zuladen eines mit dem flüssigen Mittel getränkten Trägers mit großer Oberfläche, wobei das Avivagemittel während des Trocknungsvorganges auf das Waschgut aufzieht. Gegenwärtig wird jedoch die Wäsche zum größten Teil hängend getrocknet; es überwiegt daher auch die Anwendung des Avivagemittels im letzten Spülbad des Waschprozesses bei der Hand-und insbesondere bei der Maschinenwäsche. Hierfür werden solche Mengen des erfindungsgemäßen Mittels in der Spülflotte aufgelöst, daß die Konzentration der Verbindungen der Formel I 0,05 bis 1,5 g/l, vorzugsweise 0,1 bis 1,0 g/l beträgt.

Als Verdünnungsmittel für flüssige Textilbehandlungsmittel kommen Wasser und/oder wasserlösliche organische Lösungsmittel in Frage. Brauchbare wasserlösliche organische Lösungsmittel sind die niederen Alkohole mit 1 bis 5 Kohlenstoffatome, wie Methanol, Ethanol, Propanol, Isopropylalkohol, n-Butanol, Isobutanol, ferner Aceton und Metylethylketon sowie Ethylenglycol, Propylenglycol, Diethylenglycol und deren Mono-oder Dietherderivate, insbesondere solche mit Methyl-und Ethylresten.

Die Verbindungen der allgemeinen Formel I eignen sich auch zur Herstellung von schüttfähigen Textilnachbehandlungsmitteln in Pulverform. Als Verdünnungsmittel für derartige pulverförmige Mittel eignen sich beispielsweise Harnstoff, Acetamid, Natriumsulfat und gegebenenfalls vorhandene andere übliche Bestandteile, sofern diese in fester Form vorliegen.

Als sonstige übliche Bestandteile von Textilnachbehandlungsmitteln gelten beispielsweise Dispergatoren, optische Aufheller, antimikrobielle Wirkstoffe, saure Zusatzstoffe, Lösungsvermittler, Farb-und Duftstoffe.

Die Verbindungen der allgemeinen Formel I können auch zusammen mit bekannten Avivagemitteln vom Typ der quartären Ammoniumverbindungen, wie z.B. Ditalgalkyldimethylammoniumchlorid, oder mit bekannten Avivagemitteln mit Amidstruktur vom Typ der Fettsäurekondensationsprodukte mit Hydroxyalkylpolyaminen, beispielsweise dem Kondensationsprodukt aus 1 Mol gehärtetem Talg und ein Mol 2-Hydroxyethylethylendiamin, kombiniert werden.

Die Zusammensetzung praktisch besonders interessanter flüssiger Wäschenachbehandlungsmittel, in denen die neuen Verbindungen der allgemeinen Formel I verwendet werden, liegt im allgemeinen im Bereich der folgenden Rezeptur:

| | |
|---|---|
| 2,0 - 30,0, | vorzugsweise 5,0 bis 25,0 Gew.-% Verbindungen der allgemeinen Formel I |
| 0,2 - 6,0, | vorzugsweise 0,5 bis 3,0 Gew.-% Dispergatoren, |
| 0,0 - 30,0, | vorzugsweise 1,0 bis 15,0 Gew.-% wasserlösliche organische Lösungsmittel, |
| 0,0 - 10,0, | vorzugsweise 0,3 bis 7,5 Gew.-% sonstige übliche Bestandteile von Wäschenachbehandlungsmitteln, |
| Rest | Wasser. |

Aus den sonstigen üblichen Bestandteilen von Wäschenachbehandlungsmitteln kann wenigstens eine der folgenden Komponenten in den angegebenen, auf das gesamte Mittel bezogenen Mengen vorhanden sein:

| | |
|---|---|
| 0,2 - 5,0 | Gew.-% anderer, von den Verbindungen der allgemeinen Formel I verschiedener Avivagemittel, |
| 0,2 - 3,0 | Gew.-% antimikrobielle Wirkstoffe, |
| 0,2 - 6,0 | saurer Zusatzstoff, |
| 0,01 - 0,5 | Gew.-% Baumwollaufheller, |
| 0,01 - 0,5 | Gew.-% Polyamidaufheller, |
| 0,01 - 0,5 | Gew.-% Duftstoff, |
| 0,00001 - 0,05 | Gew.-% Farbstoff. |

Im folgenden werden die in den Textilnachbehandlungsmitteln neben den Verbindungen der allgemeinen Formel I noch enthaltenen Bestandteile nach Substanzklassen geordnet näher beschrieben.

Als Dispergatoren, wie sie in flüssigen und pulverförmigen Textilnachbehandlungsmitteln enthalten sein können, eignen sich insbesondere die nichtionischen Tenside ("Nonionics"). Dazu gehören Produkte, die ihre hydrophilen Eigenschaften der Anwesenheit von Polyetherketten, Aminoxid-, Sulfoxid-oder Phosphinoxid gruppen, Alkylolamidgruppierungen sowie ganz allgemein eine Häufung von Hydroxylgruppen verdanken. Deratige Nonionics enthalten im Molekül wenigstens einen hydrophoben Rest mit 8 bis 26, vorzugsweise 10 bis 20 und insbesondere 12 bis 18 Kohlenstoffatomen, und wenigstens eine nichtionische wasserlöslichmachende Gruppe. Der vorzugsweise gesättigte hydrophobe Rest ist meist aliphatischer, gegebenenfalls auch alicyclischer Natur; er kann mit den wasserlöslichmachenden Gruppen direkt oder über Zwischenglieder verbunden sein. Als Zwischenglieder kommen z. B. Benzolringe, Carbonsäureester-oder Carbonamidgruppen, ether-oder esterartig gebundene Reste mehrwertiger Alkohole, wie z.B. die des Ethylenglycols, des Propylenglycols, des Glycerins oder entsprechender Polyetherreste in Frage.

Von besonderem praktischen Interesse sind die durch Anlageung von Ethylenoxid und/oder Glycid an Fettalkohole, Alkylphenole, Fettsäuren, Fettamine, Fettsäure-oder Sulfonsäureamide erhältlichen Produkte, wobei diese Nonionics 4 bis 100, vorzugsweise 6 bis 40 und insbesondere 8 bis 20 Etherreste, vor allem Ethylenglycoletherreste pro Molekül enthalten können. Außerdem können in diesen Polyetherketten bzw. an deren Ende Propylen-oder Butylenglycoletherreste bzw. -polyetherketten vorhanden sein. Weiterhin zählen zu den Nonionics Produkte, die man aus an sich wasserunlöslichen Polypropylenglycolen oder aus wasserunlöslichen propoxylierten niederen, 1 bis 8, vorzugsweise 3 bis 6 Kohlenstoffatome enthaltenden aliphatischen Alkoholen oder aus wasserunlöslichen propoxylierten Alkylendiaminen erhält, indem man diese bis zur Wasserlöslichkeit ethoxyliert.

Zu den Nonionics gehören auch Fettsäure-oder Sulfonsäurealkylolamine, die sich z.B. vom Mono-oder Diethanolamin, vom Dihydroxypropylamin oder anderen Polyhydroxyalkylaminen, z.B. den Glycaminen ableiten. Sie lassen sich durch Amide aus höheren primären oder sekundären Alkylaminen oder Polyhydroxycarbonsäuren ersetzen.

Weiterhin sind als brauchbare Nonionics kapillaraktive Aminoxide zu nennen wie z.B. die von höheren tertiären, einen hydrophoben Alkylrest und zwei kürzere, bis zu je 4 Kohlenstoffatome enthaltenden Alkyl-und/oder Alkylolrest aufweisenden Aminen abgeleiteten Produkte.

Außer den meist gut wasserlöslichen Tensiden eignen sich als nichtionische Dispergatoren gegebenenfalls auch wasserlösliche oder in Wasser emulgierbare oder dispergierbare Verbindungen, die entweder keine hydrophoben Reste im Sinne der oben beschriebenen nichtionischen Tenside enthalten, oder bei denen die Natur oder die Anzahl der hydrophilen Gruppen zum Erreichen einer vollständigen Wasserlöslichkeit nicht ausreichen. Zu den ersteren gehören z.B. feste oder flüssige Polyethylenglycole, die man als Kondensationspolymere des Ethylenoxids mit Ethylenglycol oder Wasser auffassen kann, sowie Ethylenoxidaddukte von Glycerin und anderen Polyalkoholen; zu den letzteren zählen z.B. Fettsäureteilglyceride oder nicht bzw. nicht vollständig wasserlösliche Alkoxylierungsprodukte, z.B. solche mit 2 bis 5 Ethylenglycoletherresten im Molekül.

Unter sauren Zusatzstoffen werden anorganische und nichtgrenzflächenaktive organische Säuren mit 2 bis 8 Kohlenstoffatomen, wie z.B. Amidosulfonsäure, Harnstoffverbindungen der Orthophosphorsäure, Borsäure, Oxalsäure, Milchsäure, Glycolsäure, Citronensäure, Weinsäure, Benzolsäure, Phtalsäure, Glukonsäure, Essigsäure und Propionsäure sowie die Benzol-, Toluol-oder Xylolsulfonsäuren, Sulfoessigsäure oder Sulfobenzzoesäure bzw. saure Alkalisalze diese Säure verstanden. Glycolsäure und Citronensäure sind wegen ihrer leichten Zugänglichkeit und Ungiftigkeit bevorzugte Säuren.

Unter antimikrobiellen Wirkstoffen werden hier bakterizid oder bakteriostatisch bzw. fungizid oder fungistatisch wirkende Verbindungen verstanden. Diese Wirkstoffe sollen entweder als solche oder in Form ihrer Salze wasserlöslich sein. Als antimikrobielle Zusätze eignen sich vor allem quartäre Ammoniumverbindungen, insbesondere solche, die neben einem langkettigen aliphatischen und zwei kurzkettigen aliphatischen Kohlenwasserstoffresten einen aromatischen, über ein aliphatisches Kohlenstoffatom mit dem Stickstoffatom verknüpften, oder einen aliphatischen, Doppelbindungen aufweisenden organischen Rest im Molekül enthalten. Beispiele für derartige antimikrobielle quartäre Ammoniumverbindungen sind die folgenden: Dimethyl-benzyl-dodecylammoniumchlorid, Dimethyl-benzyl-tetradecylammoniumchlorid, Dimethyl-(ethylbenzyl)-dodecyl-ammoniumchlorid, Dimethyl-benzyl-decylammoniumbromid, Diethyl-benzyl-dodecylammoniumchlorid, Diethyl-benzyl-octyl-ammoniumchlorid, Dibutyl-allyl-, Methyl-ethyl-benzyl-, Ethyl-cyclohexyl-allyl-und Ethyl-crotyl-diethylaminoethyl-dodecyl-ammoniumchlorid.

Weitere brauchbare antimikrobielle Wirkstoffe sind die sowohl durch Brom als auch durch die Nitrogruppe substituierten niederen Alkohole bzw. Diole mit 3 bis 5 Kohlenstoffatomen wie z.B. die Verbindungen 2-Brom-2-nitro-propan-1,3-diol, 1-Brom-1-nitro-3,3,3-trichlor-2-propanol und 2-Brom-2-nitro-butanol.

Als antimikrobielle Wirkstoffe eignen sich auch phenolische Verbindungen vom Typ der halogenierten Phenole mit 1 bis 5 Halogensubstituenten, insbesondere chlorierte Phenole; Alkyl-, Cycloalkyl-, Aralkyl-und Phenylphenole mit 1 bis 12 Kohlenstoffatomen in den Alkylresten und mit 1 bis 4 Halogensubstituenten, insbesondere Chlor und Brom im Molekül; Alkylbisphenole, insbesondere durch 2 bis 6 Halogenatome und gegebenenfalls niedere Alkyl-oder Trifluormethylgruppen substituierte Derivate, mit einem Alkylenbrückenglied bestehend aus 1 bis 10 Kohlenstoffatomen; Hydroxybenzoesäuren bzw. deren Ester und Amide, insbesondere Anilide, die im Benzoesäure-und/oder Anilinrest insbesondere durch 2 oder 3 Halogenatome und/oder Trifluormethylgruppen substituiert sein können; o-Phenyloxyphenole, die durch 1 bis 7, vorzugsweise 2 bis 5 Halogenatome und/oder die Hydroxy-, Cyano-, Carbomethoxy-und Carboxylgruppe oder niederes Alkyl substituiert sein können; ein besonders bevorzugtes Derivat des o-Phenoxyphenols ist der 2-Hydroxy-2',4,4'-trichlordiphenylether.

Unter antimikrobiellen Wirkstoffen im weiteren Sinne werden auch Zusätze, wie z.B. Formaldehyd, Sorbinsäure und Natriumfluorid verstanden, die zur Konservierung der Präparate dienen.

Die Erfindung wird durch die nachfolgenden Beispiele näher erläutert.

Beispiel 1

Herstellung von oxalkylierten 2-Alkyl-2-imidazoliniumsalzen der allgemeinen Formel (I)

In einer 2 l-Glasapparatur, die mit Rührer, Wasserabscheider, Thermometer und einem Gaseinleitungsrohr ausgerüstet war, wurden 478,4 g (4,6 mol) Aminoethylethanolamin, 1,3 g hyperphosphorige Säure (50 %ig) und 150 ml Xylol vorgelegt und mit 824,0 g (4,0 mol) Kokosfettsäure (allgemeine Formel (II), $R^1$ = Alkylrest mit 8 bis 18 C-Atomen) gemischt. Danach wurde unter Einleiten von Stickstoff bis zu einer Sumpftemperatur von 208°C zum Rückfluß erhitzt. Nachdem 165 g aminhaltiges Wasser abgeschieden waren, wurden bis zu einem Enddruck von 20 mbar und einer Sumpftemperatur von 220°C Xylol und überschüssiges Amin abdestilliert. Man erhielt 1098 g einer hellgelben, öligen Flüssigkeit, die nach einiger Zeit erstarrte.

## Tabelle 1

| lfd. Nr. | Basisfettsäure für A $R^1COOH$ | Alkylenoxid (mol) | Lösungsmittel (%) $H_2O$ | Lösungsmittel (%) PG* | Säurezahl vor Umsetzung | Säurezahl nach Umsetzung | Umsetzungsgrad (%) aus **Z.Titr. | Umsetzungsgrad (%) aus S.Z. | Konsistenz des Produktes |
|---|---|---|---|---|---|---|---|---|---|
| 1 | Kokosfetts. C8/18 | 3 EO | 11,5 | 8,0 | 89,9 | 13,7 | 82,0 | 84,8 | homogene, niedrig-viskose Flüssigkeit |
| 2 | Laurinsäure | " | 12,2 | 9,0 | 90,1 | 19,0 | 77,5 | 78,9 | 50 %ig sehr niedrig viskos |
| 3 | Kokosfetts. C8/18 | " | 20 | - | 89,4 | 15,1 | - | 83,1 | 40 %ig sehr niedrig viskos |
| 4 | " | 5 EO | 20 | - | 96,3 | 2,8 | - | 96,3 | 50 %ig sehr niedrig viskos |
| 5 | " | 3 PO | 20 | - | 82,5 | 17,9 | - | 78,3 | homogene, niedrig-viskose Flüssigkeit |

* Propylenglykol-1,2

** Zweiphasentitration

Analysenwerte:

| | |
|---|---|
| Stickstoff (gesamt) | 10,1 % |
| Stickstoff (basisch) | 5,0 % |
| Imidazolin (UV-spektrometrisch) | 98 % |
| Diamid (Austauscherdurchlauf) | 1,5 % |

In einem Rührautoklaven wurden nun 82,5 g Wasser und 60,0 g (0,6 mol) einer 90 %igen Milchsäure vorgelegt und mit 168,0 g (0,6 Aminäquivalente) des wie oben beschrieben erhaltenen Imidazolins gemischt. Der Autoklav wurde verschlossen und die Luft durch Spülung mit Stickstoff verdrängt.

Danach wurde bei 80 bis 85°C 132,0 g (3,0 mol) Ethylenoxid in der Weise zugegeben, daß ein Druck von 3 bar nicht überschritten wurde. Nach beendeter Zugabe ließ man noch 4 h bei 80 bis 85°C nachreagieren.

Es wurden 440,2 g einer gelben, niedrigviskosen, klaren Flüssigkeit erhalten.

Säurezahl: 2,8

(die rechnerische Säurezahl des Gemisches vor der Reaktion würde 76,1 betragen). Daraus resultiert ein Umsatz von 96,3 % zur quartären Ammoniumverbindung.

Eine HPLC-Analyse ergab einen Ethylenglykolgehalt von 14,2 % und 0,38 % Diethylenglykol.

In analoger Weise wurden auch ausgehend von anderen Basisfettsäuren oxalkylierte Imidazoliniumsalze der allgemeinen Formel (I) hergestellt. Die Ergebnisse sind der nachfolgenden Tabelle 1 zu entnehmen.

Beispiel 2

Herstellung weiterer Verbindungen der allgemeinen Formel (I) gemäß der Erfindung

In einem 4 l-Glaskolben, der mit Rührer, absteigendem Liebigkühler, Thermometer und Gaseinleitungsrohr ausgerüstet war, wurden 2189,6 g (8,0 mol) einer technischen Ölsäure und 2,5 g hyperphosphorige Säure (50 %ig) vorgelegt. Die Reaktionsmischung wurde unter Einleiten von Stickstoff bei 65°C mit 412 g (4,0 mol) Diethylentriamin versetzt. Während des darauffolgenden dreistündigen Aufheizens auf 200°C unter Einleiten von Stickstoff begann bei 148°C eine kräftige Wasserabspaltung. Nach einer Verweilzeit von 1 h bei 200°C waren insgesamt 154 g Destillat angefallen. Der Reaktionsraum wurde anschließend allmählich bis zu einem Enddruck von 20 mbar evakuiert, wobei weiteres Destillat gesammelt wurde. Unter Beibehaltung des Vakuums wurde noch 1 h bei 210 bis 220°C nachgerührt.

Es wurden 2380 g einer gelben, mittelviskosen Flüssigkeit erhalten.

Analysendaten:

| | |
|---|---|
| Stickstoff (gesamt) | 7,0 % |
| Stickstoff (basisch) | 2,35 % |
| Imidazolin (UV-spektrometrisch) | 99,0 % |

Die Oxethylierung dieses Imidazolins erfolgte wie schon in Beispiel 1 beschrieben. Dabei wurden 66,0 g Wasser, 30,0 g (0,3 mol) Milchsäure (90 %ig), 182,6 g (0,3 Aminäquivalente) Imidazolin und 66,0 g (1,5 mol) Ethylenoxid vorgelegt.

Auf dem schon in Beispiel 1 beschriebenen Weg wurden 342 g einer Gelee-artigen Paste erhalten.

Säurezahl: 4,5

(die rechnerische Säurezahl des Gemisches vor der Reaktion würde 98,8 betragen). Daraus resultiert ein Umsatz von 90,8 % zur oxalkylierten 2-Alkyl-2-imidazoliniumverbindung der allgemeinen Formel (I).

HPLC-Analyse des Produktes:

14,3 % Ethylenglykol und

0,38 % Diethylenglykol.

Auf dem vorstehend beschriebenen Weg wurden noch weitere Verbindungen der allgemeinen Formel (I) erhalten. Die Ergebnisse sind der nachfolgenden Tabelle 2 zu entnehmen.

Tabelle 2

| lfd. Nr. | Basisfettsäure für B $R^1COOH$ | Alkylenoxid (mol) | Lösungsmittel (%) $H_2O$ | Lösungsmittel (%) PG* | Säurezahl vor Umsetzung | Säurezahl nach Umsetzung | Umsetzungsgrad (%) aus **Z.Titr. | Umsetzungsgrad (%) aus S.Z. | Konsistenz des Produktes |
|---|---|---|---|---|---|---|---|---|---|
| 6 | Ölsäure, Produkt dest. | 3 EO | 7,9 | 12,1 | 54,0 | 6,4 | 82 | 88,0 | opake, fließende Paste |
| 7 | 75 Gew.-T. Ölsäure 25 Gew.-T. Stearins. | " | 7,9 | 12,1 | 54,0 | 7,8 | - | 85,6 | talgartige Paste |
| 8 | Ölsäure | " | 20 | - | 54,0 | 14,3 | - | 73,5 | gelartige Paste |
| 9 | " | 5 EO | 20 | - | 48,8 | 4,5 | - | 90,8 | " " |
| 10 | " | 2 EO | 20 | - | 57,0 | 32,5 | - | 43,0 | " " |
| 11 | Fettsäure C8/10 u. C16/18 im Molverh. 1 : 1 | 3 EO | 20 | - | 64,4 | 11,6 | - | 82,0 | salbige " |
| 12 | Ölsäure | 3 PO | 20 | - | 51,4 | 9,8 | - | 80,9 | flüssig, leichte Separierung |
| 13 | " | 5 PO | 20 | - | 45,4 | 1,3 | - | 97,1 | homogene, niedrig viskose Flüssigkeit |
| 14 | 75 Gew.-T. Ölsäure 25 Gew.-T. Stearins. | 5 PO | 20 | - | 45,4 | 1,2 | - | 97,3 | schmalzartige Paste |
| 15 | Stearinsäure | 3 PO | 20 | - | 51,4 | 7,1 | - | 86,2 | talgartige Paste |

* Propylenglykol-1,2

** Zweiphasentitration

Beispiel 3

Anwendungstechnische Prüfung

a) Avivagewirkung

Die avivierende Wirkung der Verbindungen der allgemeinen Formel (I) wurde an Baumwollfrotteegeweben geprüft, die durch mehrfaches Waschen hartgemacht waren. Die Gewerbeproben wurden in einer wässrigen Flotte (Leitungswasser 20°dH; Flottenverhältnis 1:20), die - bezogen auf das Gewicht der Gewebe - 0,5 Gew.-% Wirksubstanz enthielt, 3 Minuten lang schwach bewegt. Danach wurden die Gewebe durch Ausdrücken weitgehend von anhaftender Behandlungsflotte befreit und an der Luft getrocknet. Die Weichheit der getrockneten Gewebe wurde von einer Gruppe von Personen haptisch beurteilt, die Erfahrungen in dieser Art von Prüfungen haben.

b) Wiederbenetzbarkeit

Die Wiederbenetzbarkeit wurde an 2 × 2 cm großen Stücken von Baumwollgeweben geprüft, die wie oben beschrieben vorbehandelt waren. Die trockenen Gewebeproben wurden bei Raumtemperatur auf eine Leitungswasseroberfläche gelegt. Mit einer Stoppuhr wurde die Zeit gemessen, nach der die Probe vollständig benetzt war und nach unten sank.

Die Prüfungen wurden mit den erfindungsgemäßen Substanzen 6, 8, 10, 11, 12 und 15 (siehe Tabelle 2) durchgeführt. Zu Vergleichszwecken wurde Distearyldimethylammoniumchlorid (A), ein handelsübliches Avivagemittel, in die Prüfung miteinbezogen.

Die gefundenen Ergebnisse sind in der nachstehenden Tabelle 3 zusammengefaßt. In dieser Tabelle sind die geprüften Substanzen so angeordnet, daß ihre avivierende Wirkung von unten nach oben zunimmt.

Tabelle 3

Avivagewirkung und Wiederbenetzbarkeit

| Substanz | Avivagewirkung | vollständig benetzt nach (sec) |
|---|---|---|
| A* | sehr gut | wesentlich mehr als 300 |
| 15 | ↑ | 2,5 |
| 11 | | 2,5 |
| 13 | | 1,0 |
| 6 | | 2,5 |
| 10 | | 1,5 |
| 8 | | 2,0 |
| 12 | gut | 1,5 |

*) Vergleichssubstanz

c) Aniontensidverträglichkeit

Zur Prüfung der Aniontensidverträglichkeit wurden die Substanzen 1 bis 5 (siehe Tabelle 1) jeweils mit Natriumalkylethersulfat auf Basis eines Anlagerungsproduktes von 2 Mol Ethylenoxid an ein Gemisch aus $C_{12}$-und $C_{14}$-Fettalkohol (Gewichtsverhältnis 70:30) im Molverhältnis Aniontensid : Kationtensid = 3:1 gemischt und aus den erhaltenen Mischungen wässrige Lö sungen mit einem Gesamttensidgehalt von 10 Gew.-% hergestellt. Zu Vergleichszwecken wurde Cetyltrimethylammoniumchlorid (B) in die Prüfung mit einbezogen. Die gefundenen Ergebnisse können der nachstehenden Tabelle 4 entnommen werden (+ = verträglich; - = unverträglich).

Tabelle 5

Aniontensidverträglichkeit

| Substanz | Verträglichkeit |
|---|---|
| 1 | + |
| 2 | + |
| 3 | + |
| 4 | + |
| 5 | + |
| B | + - (trüb) |

Ergebnis:

Die erfindungsgemäßen quartären 2-Alkyl-2-imidazoliniumsalze der allgemeinen Formel (I) sind in ihrer Avivagewirkung dem handelsüblichen Distearyldimethylammoniumchlorid (A) praktisch gleichwertig. Überraschenderweise zeigt sich jedoch, daß die mit den erfindungsgemäßen Verbindungen der Formel (I) behandelten Gewebe über eine ausgezeichnete Wiederbenetzbarkeit verfügen, die den mit dem Mittel des Standes der Technik behandelten Textilien praktisch vollkommen fehlt. Aus der Tabelle 3 ist auch ersichtlich, daß sich die Avivagewirkung der neuen Verbindungen durch Art und Grad der Alkoxylierung einstellen läßt. Weiterhin besitzen die erfindungsgemäßen Verbindungen gegenüber Aniontensiden eine sehr gute Verträglichkeit.

**Ansprüche**

1. Quartäre 2-Alkyl-2-imidazoliniumsalze der allgemeinen Formel (I)

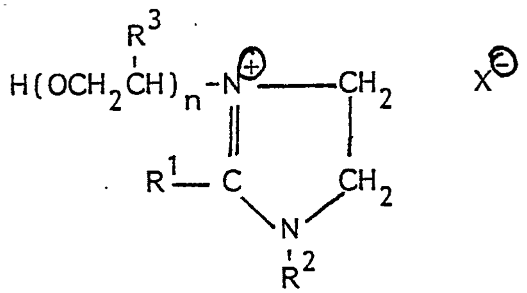

(I)

in der
$R^1$ für einen geradkettigen oder verzweigten, substituierten oder unsubstituierten Alkyl-oder Alkenylrest mit 7 bis 21 C-Atomen,
$R^2$ für einen Rest der Strukturen

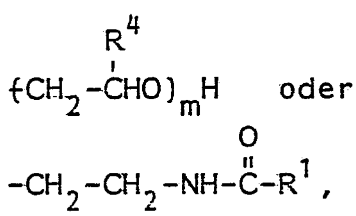

oder

$R^3$ für Wasserstoff oder Methyl,
$R^4$ für Wasserstoff oder Methyl
$X^\ominus$ für das Anion einer nicht oxidierend und nicht korrosiv wirkenden anorganischen Säure oder einer organischen Mono-oder Polycarbonsäure und
n statistisch für ganze oder gebrochene Zahlen im Bereich von 1 bis 20 und
m für ganze Zahlen im Bereich von 1 bis 3 stehen.

2. Salze der allgemeinen Formel (I) nach Anspruch 1, dadurch gekennzeichnet, daß $R^1$ für einen geradkettigen oder verzweigten, unsubstituierten Alkyl-oder Alkenylrest mit 7 bis 17 C-Atomen steht.

3. Salze nach Ansprüchen 1 und 2, dadurch gekennzeichnet, daß in der allgemeinen Formel (I) $R^1$ für einen Alkylrest aus der Gruppe n-Heptyl, n-Nonyl, n-Undecyl, n-Tridecyl, n-Pentadecyl, n-Heptadecyl, n-Heptadec-8-enyl, n-Heptadec-8,11-dienyl oder deren Mischungen steht.

4. Salze nach Anspruch 1, dadurch gekennzeichnet, daß in der allgemeinen Formel (I) $R^1$ für einen geradkettigen oder verzweigten, mit einer oder mehreren Hydroxylgruppen substituierten Alkylrest mit 7 bis 17 C-Atomen steht.

5. Salze nach Ansprüchen 1 bis 4, dadurch gekennzeichnet, daß in der allgemeinen Formel (I) $R^2$ für einen Hydroxyethylrest steht.

6. Salze nach Ansprüchen 1 bis 5, dadurch gekennzeichnet, daß in der allgemeinen Formel (I) $X^\ominus$ für das Anion einer Alkancarbonsäure mit 0 bis 17 C-Atomen im Alkylrest, das Anion einer Monohydroxyalkancarbonsäure mit 1 bis 17 C-Atomen im Alkylrest oder das Anion einer $\alpha,\omega$-Dicarbonsäure mit 0 bis 8 Methylengruppen, wobei die Methylengruppen einen Hydroxysubstituenten tragen können, oder das Anion der Fumarsäure, Maleinsäure oder Citronensäure stehen kann.

7. Salze nach Anspruch 6, dadurch gekennzeichnet, daß in der allgemeinen Formel (I) das Anion $X^\ominus$ ein Anion der Milchsäure oder Essigsäure ist.

8. Salze nach Ansprüchen 1 bis 7, dadurch gekennzeichnet, daß in der allgemeinen Formel (I) n statistisch für ganze oder gebrochene Zahlen im Bereich von 1 bis 10 steht.

9. Salze nach Ansprüchen 1 bis 8, dadurch gekennzeichnet, daß in der allgemeinen Formel (I) $R^3$ für Wasserstoff steht.

10. Verfahren zur Herstellung von quartären 2-Alkyl-2-imidazoliniumsalzen der allgemeinen Formel (I)

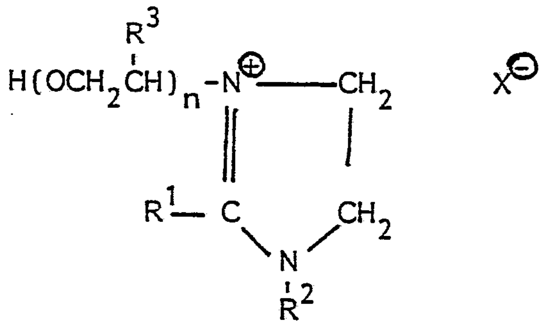

(I)

in der

$R^1$ für einen geradkettigen oder verweigten, substituierten oder unsubstituierten Alkyl-oder Alkenylrest mit 7 bis 21 C-Atomen,

$R^2$ für einen Rest der Strukturen

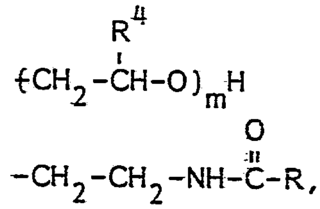

oder

$R^3$ für Wasserstoff oder Methyl,

$R^4$ für Wasserstoff oder Methyl,

$X^\ominus$ für das Anion einer nicht oxidierend und nicht korrosiv wirkenden anorganischen Säure oder einer organischen Mono-oder Polycarbonsäure und

n statistisch für ganze oder gebrochene Zahlen im Bereich von 1 bis 20 und

m für ganze Zahlen im Bereich von 1 bis 3 stehen, dadurch gekennzeichnet, daß man Imidazoline der allgemeinen Formel (II)

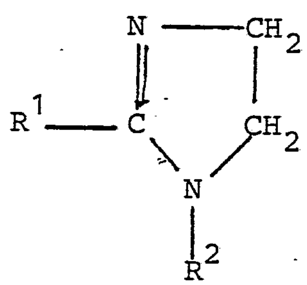

(II)

in der $R^1$ und $R^2$ die für die Formel (I) angegebene Bedeutung haben, in Gegenwart von 1 bis 1,1 Äquivalenten einer Säure der Formel HX (III) in der X die für die Formel (I) angegebene Bedeutung hat, und gegebenenfalls in Gegenwart von Wasser oder eines Gemisches aus Wasser und einem polaren organischen Lösungsmittel in an sich bekannter Weise mit Ethylenoxid oder Propylenoxid umsetzt und die entstandenen Produkte gegebenenfalls auf an sich bekannten Wegen isoliert und/oder reinigt.

11. Verfahren nach Anspruch 10, dadurch gekennzeichnet, daß in den Formeln (I) und (II) $R^1$ für einen geradkettigen oder verzweigten unsubstituierten Alkylrest mit 7 bis 17 C-Atomen steht.

12. Verfahren nach Anspruch 11, dadurch gekennzeichnet, daß in den Formeln (I) und (II) $R^1$ für einen Alkylrest aus der Gruppe n-Heptyl, -n-Nonyl, n-Undecyl, n-Tridecyl, n-Pentadecyl, n-Heptadecyl, n-Heptadec-8-enyl, n-Heptadec-8,11-dienyl oder deren Mischungen steht.

13. Verfahren nach Anspruch 11, dadurch gekennzeichnet, daß in den Formeln I und II $R^1$ für einen mit einer oder mehreren Hydroxylgruppen substituierten Alkylrest steht.

14. Verfahren nach den Ansprüchen 10 bis 13, dadurch gekennzeichnet, daß in den Formeln (I) und (II) $R^2$ für einen Hydroxyethylrest steht.

15. Verfahren nach den Ansprüchen 10 bis 13, dadurch gekennzeichnet, daß in den Formeln (I) und (II) $R^2$ für einen $-CH_2-CH_2-NH-CO-R^1$-Rest steht.

16. Verfahren nach den Ansprüchen 10 bis 15, dadurch gekennzeichnet, daß in den Formeln (I) und (III) X für das Anion einer Alkancarbonsäure mit 0 bis 17 C-Atomen im Alkylrest das Anion einer Monohydroxyalkancarbonsäure mit 1 bis 17 C-Atomen im Alkylrest oder das Anion einer $\alpha,\omega$-Dicarbonsäure mit 0 bis 8 Methylengruppen, wobei die Methylengruppen Hydroxysubstituenten tragen können, oder das Anion der Fumarsäure, Maleinsäure oder Citronensäure steht.

17. Verfahren nach den Ansprüchen 10 bis 15, dadurch gekennzeichnet, daß in den Formeln (I) und (III) X für das Anion der Milchsäure oder Essigsäure steht.

18. Verfahren nach den Ansprüchen 10 bis 17, dadurch gekennzeichnet, daß man die Umsetzung in Gegenwart eines Gemisches aus Wasser und mindestens einem Alkohol mit 1 bis 6 C-Atomen durchführt.

19. Verfahren nach Anspruch 18, dadurch gekennzeichnet, daß man die Umsetzung in Gegenwart eines Gemisches aus Wasser und Isopropanol im Volumenverhältnis 1 : 1 durchführt.

20. Verfahren nach den Ansprüchen 10 bis 19, dadurch gekennzeichnet, daß man die Umsetzung in einem Molverhältnis von Imidazolin : Alkylenoxid von 1 : 1 bis 1 : 20 durchführt.

21. Verfahren nach Anspruch 20, dadurch gekennzeichnet, daß man die Umsetzung in einem Molverhältnis von 1 : 3 bis 1 : 10 durchführt.

22. Verfahren nach den Ansprüchen 10 bis 21, dadurch gekennzeichnet, daß man die Umsetzung mit Ethylenoxid durchführt.

23. Verwendung von quartären 2-Alkyl-2-imidazoliniumsalzen der allgemeinen Formel (I)

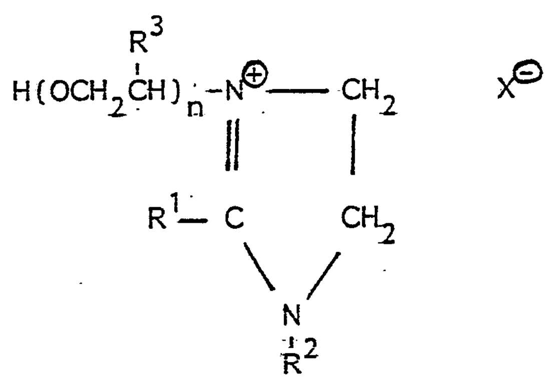

(I)

in der

$R^1$ für einen geradkettigen oder verweigten, substituierten oder unsubstituierten Alkyl-oder Alkenylrest mit 7 bis 21 C-Atomen,

$R^2$ für einen Rest der Strukturen

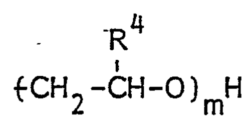

oder

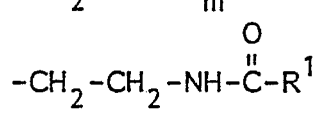

,

$R^3$ für Wasserstoff oder Methyl,

$R^4$ für Wasserstoff oder Methyl,

$X^\ominus$ für das Anion einer nicht oxidierend und nicht korrosiv wirkenden anorganischen Säure oder einer organischen Mono-oder Polycarbonsäure und

n statistisch für ganze oder gebrochene Zahlen im Bereich von 1 bis 20 und

m für ganze Zahlen im Bereich von 1 bis 3

stehen, zur Avivage von Textilien.

Europäisches Patentamt

# EUROPÄISCHER RECHERCHENBERICHT

Nummer der Anmeldung

EP 87107816.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US - A - 4 127 489 (PRACHT et al.)<br>* Ansprüche; Beispiel 1 *<br>-- | 1,10, 23 | C 07 D 233/14<br>C 07 D 233/18<br>C 07 D 233/22<br>D 06 M 13/46 |
| A | DE - A1 - 2 930 849 (HENKEL KG & A)<br>* Ansprüche 1,2 *<br>-- | 1,10, 23 | |
| A | EP - A1 - 0 075 170 (BAYER AG)<br>* Zusammenfassung *<br>-- | 1,10, 23 | |
| A | AT - B - E 4 334 (THE PROCTER & GAMBLE COMPANY)<br>* Ansprüche *<br>-- | 1,2,3 | |
| A | EP - A2 - 0 056 695 (PROCTER & GAMBLE EUROPEAN TECHNICAL CENTER)<br>* Anspruch 1; Seite 13, Zeile 19 - Seite 14, Zeile 3 *<br>-- | 1,2,3 | **RECHERCHIERTE SACHGEBIETE (Int. Cl.4)** |
| A | GB - A - 2 167 092 (COLGATE-PALMOLIVE COMPANY)<br>* Ansprüche 1-10; Seite 1, Zeile 57 - Seite 2, Zeile 38 *<br>-- | 1,2,3 | C 07 D 233/00<br>D 06 M 13/00 |
| A | CH - A5 - 639 444 (THE PROCTER & GAMBLE COMPANY)<br>* Ansprüche *<br>-- | 1,2,3 | |
| A | US - A - 4 526 694 (PUCHTA et al.)<br>* Zusammenfassung; Spalten 6,7, 36-42 *<br>---- | 1,10, 23 | |

Der vorliegende Recherchenbericht wurde für alle Patentansprüche erstellt.

| Recherchenort | Abschlußdatum der Recherche | Prüfer |
|---|---|---|
| WIEN | 14-09-1987 | BRUS |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument